# EUROPEAN PATENT SPECIFICATION

(11) **EP 1 680 070 B1**
(45) Date of publication and mention of the grant of the patent: **23.04.2008**
(21) Application number: 04751638.0
(22) Date of filing: 07.05.2004
(51) Int. Cl.: A61K 8/06, A61K 8/81, A61Q 19/00, A61Q 1/14, A61Q 17/04, A61Q 19/04, A61Q 1/00, A61Q 5/12

(54) **EMULSIFIERS FOR MULTIPLE EMULSIONS**
EMULGATOR FÜR MEHRPHASIGE EMULSIONEN
EMULSIFIANTS POUR EMULSIONS MULTIPLES

(30) Priority: 07.05.2003 US 468439 P
(43) Date of publication of application: 19.07.2006
(73) Proprietor: The Lubrizol Corporation, Wickliffe, Ohio 44092-2298 (US)
(72) Inventor: Filippini, Brian B., Mentor-on-the-Lake, OH 44060 (US); HUANG, Nai, Z., Highland Hts., OH 44143 (US); CAREY, Jeffrey, M., Mentor, OH 44060 (US); MULLAY, John, J., Mentor, OH 44060 (US)
(74) Representative: Weber, Thomas
(86) International application number: PCT/US2004/014336
(87) International publication number: WO 2004/100904

(56) References cited:
- EP-A- 0 715 842
- EP-A- 0 717 978
- US-A- 4 256 605
- US-A- 5 034 071
- US-A- 5 457 835

## Description

This application claims priority from U.S. provisional application Serial Number 60/468,439 filed May 7, 2003, now pending.

### Field of the Invention

The present invention relates to a multiple emulsion, in particular a water-in-oil-in-water emulsion (WOW). The multiple emulsions are particularly useful in industrial, household and consumer products. Additionally, the multiple emulsions are useful in personal care products.

### Background of the Invention

The human need to look and feel satisfied with their personal appearance has provided growth to the personal care industry over the last several years. There are several different types of creams and lotions that can be used that contain several different types of emulsified compositions in order to obtain the desired results.

A personal care product in the form of an emulsion may be made to the necessary and desired viscosity that is from a fluid to less fluid to a soft paste. An emulsified product has the further advantage of being pleasant in appearance and easy to apply.

The majority of personal care products are in the form of emulsions that is water-in-oil (W/O) or oil-in-water (O/W), which allows the simultaneous use of lipophilic and hydrophilic ingredients in the required quantities. An emulsion is defined physically as a liquid system with two fluids that are insoluble or only sparingly soluble in each other, and in which one phase is finely dispersed within the other. Personal care product emulsions generally consist of a polar water phase and a nonpolar oil phase and are either oil-in-water emulsions or water-in-oil emulsions. The necessary stabilization of the emulsion is achieved during production by the introduction of an emulsifier which reduces the interfacial tension between the two phases. Emulsions are of great importance for personal care products, as they meet the physiological demands of the skin and permit uniform distribution of water soluble and oil soluble substances.

An obvious disadvantage in the use of emulsions is that the materials to be combined are not inherently compatible. The tendency is for the water and oil to separate.

The problem is further magnified in the formulation of desired multiple emulsions that is water-in-oil-in-water ("WOW') or oil-in-water-in-oil ("OWO") emulsions. Multiple WOW emulsions are systems formed from tiny drops of oil dispersed in water, but at the same time having even tinier drops of water encapsulated within the emulsified oil. In practical terms, a drop of oil contains other minute particles of water, and is suspended in water. In an emulsion of this type, there is a coexistence of a W/O emulsion in an O/W emulsions. Naturally, it is possible to create the opposite; that is OWO emulsion system. Multiple emulsions allow the encapsulation, within the same product, of non-compatible active ingredients.

Such multiple emulsions generally have not been practical, because they tended to destabilize because of the presence of two incompatible emulsifiers in one system. This instability is exacerbated in those cases in which either the middle oil phase or the external water phase has a relatively low viscosity. In these cases either greater coalescence can occur in the internal water-in-oil emulsion or greater settling or creaming can occur in the overall emulsion. This in turn leads to the need to add ingredients that provide greater viscosity and also the use of large amounts of emulsifiers in order to maintain required stability properties.. However, large amounts of emulsifiers and/or consistency or viscosity modification factors can detract from the desired properties of the personal care product.

Additionally, multiple emulsions have not been commercially useful because the above mentioned emulsifier incompatibility causes the internal water-in-oil emulsion and the external oil-in-water emulsion to counteract each other and collapse to either a water-in-oil or an oil-in-water emulsion. Thus, the multiple emulsions are destabilized and the product is rendered useless. It is desired to make a multiple emulsion so that the internal water-in-oil emulsion co-exists in the external aqueous phase for an acceptable period of time so as to make the overall product useful to consumers.

It is an object of this invention to provide a water-in-oil-in-water emulsion which is stable and has good emulsification properties and further can be commercially viable.

It is an object of this invention to obtain a desired product with two incompatible active ingredients through a multiple emulsion that isolates the active ingredients from each other and where the multiple emulsions is stable over time.

It has been discovered that stable water-in-oil emulsions and multiple emulsions can be made with the emulsifiers of the present invention. Further it has been discovered that a stable multiple emulsion with two or more incompatible active ingredients can be made.

### Summary of the Invention

The present invention relates to a stable multiple water-in-oil-in-water emulsion comprising:
1) A discontinuous phase, wherein that discontinuous phase is an internal water-in-oil emulsion comprising a) a water discontinuous phase, b) an oil continuous phase, c) either polyisobutenyl succinic anhydride-derived emulsifier(s) ("PIBSA") or polyisobutylene ("PIB") derived emulsifier(s) or both d) optionally a co-emulsifier and e)optionally thickeners for the discontinuous phase resulting in an internal water-in-oil emulsion; and
2) A continuous phase, wherein that continuous phase is an external aqueous phase comprising: a) water b) the internal water-in-oil emulsion, c) an oil-in-water emulsifier and c) optionally thickeners for the external aqueous phase
resulting in a stable water-in-oil-in-water multiple emulsion.

The multiple emulsion of the present invention segregates non-compatible active ingredients and allows for stable coexistence of the internal water-in-oil emulsion in the external aqueous phase. The multiple emulsion further provides good long-term storage properties.

The multiple emulsions are useful in industrial, household and consumer products. The multiple emulsions are useful in the personal care industry for dermatological, cosmetics, skin, hair, facial, sun care and the like type of products.

### Detailed Description of the Invention

The present invention relates to stable multiple emulsions. The multiple emulsion is formed from the combination of an internal phase of a water-in-oil emulsion and an external aqueous phase.

The emulsifier includes PIBSA, PIB or PIBSA derivatives, functionalized PIB or PIBSA, and combinations thereof. In one embodiment the emulsifier is the reaction product of PIBSA with triethanolamine, in another embodiment the emulsifier is the reaction product of PIBSA with glycerol, and in another embodiment the emulsifier is the reaction product of PIBSA with sodium hydroxide.

PIBSAs are made by the reaction of poly(isobutylene) with maleic anhydride, providing the poly(isobutylenyl) succinic anhydride, or PIBSA. The PIBSA may contain about 30 to about 500 carbon atoms, and in one embodiment about 50 to about 300 carbon atoms, and in one embodiment about 60 to about 200 carbon atoms. In one embodiment, the hydrocarbyl substituents of these acylating agents have number average molecular weights of about 700 to about 10000, and in one embodiment about 900 to about 2300. Desirably the mole ratio of maleic anhydride to polyisobutylene present in the reaction mixture is less than 1.3:1 and in another embodiment is 1.3:1 to 2.5:1. More desirably the mole ratio is less than 1:1 and preferably is it from about 0.6:1 to about 0.9 or 1:1.

The emulsifier can then be made by reaction of the PIBSA with a nucleophile, such as an alcohol or polyol, an amine or polyamine, an amino alcohol, or a metal-containing base such as sodium hydroxide or potassium hydroxide.

In one embodiment, polyisobutenyl substituted phenols can be used as the PIB-derived emulsifier of this invention.

In another embodiment, functionalized polyisobutenyl phenols, such as amino polyisobutenyl phenols, can be used as the PIB-derived emulsifier of this experiment. These materials can be made by (a) the reaction of an alkylphenol directly with an aldehyde and an amine resulting in an alkylphenol connected by a methylene group to an amine or (b) the reaction of an alkylphenol with an aldehyde resulting in an oligomer wherein the alkylphenols are bridged with methylene groups, and the oligomer is then reacted with more aldehyde and an amine to give a Mannich product.

The reaction products of polyisobutylene with α,β-unsaturated olefins other than maleic anhydride can be further functionalized by reaction with nucleophiles like water, alcohol, polyols, amines, polyamines, alkanolamines, inorganic bases, etc., and these reaction products can also be used as emulsifiers for multiple emulsions as well.to be used as PIB-derived emulsifier in this invention in one embodiment.

The reaction products of polyisobutylene with glyoxylic acid, lower alkyl glyoxylates, such as methyl glyoxylate, or lower alkyl hemiacetals, such acid methyl glyoxylate methyl hemiacetal, can be further functionalized with primary or secondary amines, primary or secondary alkanolamines, or polyamines, and these reaction products can also be used as the PIB-derived emulsifier in this invention.

In another embodiment, polyisobutenyl amine may be used as the PIB-derived emulsifier of this invention.

The emulsifier in the water-in-oil emulsion is used in the range from about 0.1 wt.% to about 30 wt.%, in another embodiment in the range from about 0.3 wt.% to about 10 wt.% and in another embodiment in the range from about 0.5 wt.% to about 8 wt.% of the final multiple emulsion.

The use of PIB or PIBSA and their derivatives as emulsifiers makes it possible to prepare water-in-oil emulsions that constitute stable resistant and tolerant emulsions that remain intact when blended into an external aqueous phase that contains an oil-in-water emulsifier.

The coemulsifier includes any emulsifier that will give a water-in-oil emulsion when used in combination with a PIB or PIBSA derived emulsifier. The two emulsifiers together should have a HLB value of 0-10 depending on the specific oil phase being used. The co-emulsifier includes sorbitan monooleate, sorbitan monoisostearate, glycerol monooleate, oleyl alcohol 2-ethoxylate, lecithin, sorbitan monolaurate, sorbitan monopalmitate, sorbitan monostearate, sorbitan sesquistearate, sorbitan trioleate, stearyl alcohol 2-ethoxylate, glycerol monostearate, sorbitan dioleate, refined wool fat, methyl glucoside dioleate, polyglyceryl-3 diisostearate, polyethylene glycol 200 distearate, methyl glucose sesquistearate, polyethylene glycol 200 monostearate and the like. In addition other alcohol alkoxylates as well as copolymers of various alkoxylates or ethoxylated amines and/or amides or betaines. Ionic surfactants can also be used in combination with the primary emulsifier described above as long as compatibility issues are not encountered and that a water-in-oil emulsion is produced. The coemulsifier can be used alone or in combination.

However, any type of emulsifier meeting the HLB requirement can be used. Examples of other emulsifiers of this type can be found in McCutcheon's, Vol 1:Emulsifiers & Detergents, 2000.

The coemulsifier is present in the range from about 0 wt.% to about 10 wt.%, in one embodiment in the range from about 0.05 wt.% to about 8 wt.%, and in another embodiment in the range from about 0.2 wt.% to about 5 wt.% and of the internal water-in-oil emulsion.

The aqueous phase may be any acceptable water based material and includes tap water, demineralized water, deionized water, floral water or the like. The water may be used alone or in combination.

The water is present in the range from about 1 wt.% to about 99 wt.%, in one embodiment in the range from about 20 wt.% to about 90 wt.% and in another embodiment in the range from about 20 wt.% to about 80 wt.% of the internal phase of the water-in-oil emulsion.

In the present case, the water and oil components and/or additives can be any of the standard components that are ordinary used for the desired emulsified product.

The oil phase includes all fatty substances conventionally used in the applications field, and in particular oils. The oils may be volatile or non-volatile, or a mixture of both. The oils may be used alone or in combinations.

Suitable volatile oils include, both cyclic and linear silicones, such as octamethylcyclotetrasiloxane and decamethylcyclopentasiloxane; or straight or branched chain hydrocarbons having from 8-20 carbon atoms, such as decane, dodecane, tridecane, tetradecane, squalane, hydrogenated polyisobutene, isohexadecane, or isoparaffins.

Non-volatile oils include, vegetable oils, such as apricot stone, avocado oil, macadamia nut oil, olive oil, coconut oil, jojoba oil, corn oil, sunflower oil, palm oil, soybean oil; carboxylic acid esters such as isostearyl neopentanoate, cetyl octanoate, cetyl ricinoleate, octyl palmitate, dioctyl malate, coco-dicaprylate/caprate, decyl isostearate, myristyl myristate; animal oils such as lanolin, lanolin derivatives, tallow, mink oil, cholesterol; glyceryl esters, such as glyceryl stearate, glyceryl myristate; non-volatile silicones, such dimethicone, dimethiconol, dimethicone copolyol, phenyl trimethicone, methicone, simethicone; nonvolatile hydrocarbons, such as isoparaffins, squalane; petroleum; mineral oils such as liquid paraffin; white oil, synthetic oils such as hydrogenated polyisobutene, esters of fatty acids and fatty alcohols (as C₆-C₃₀), ethers of fatty alcohols (as saturated and/or branched C₄-C₃₀ fatty alcohols); silicone oils, such as volatile cyclomethicone oils, such as cyclotetradimethylsiloxane, cyclopentamethicone, cyclohexadimethylsiloxane or cyclohexamethicone; fluorinated oils; and mixtures thereof.

The oils include 2-ethylhexyl palmitate (or octyl palmitate), 2-ethylhexyl myristate (or octyl myristate), isopropyl palmitate, isopropyl myristate, diisopropyl adipate, dioctyl adipate, 2-ethylhexyl hexanoate, ethyl laurate, methyl myristate, octyldodecyl octanoate, isodecyl neopentanoate, ethyl myristate, myristyl propionate, 2-ethylhexyl caprate/caprylate, methyl palmitate, butyl myristate, isobutyl myristate, ethyl palmitate, isohexyl laurate, hexyl laurate, isopropyl isostearate, and mixtures thereof. The oils include fatty acid esters comprising at least 12 carbon atoms. In one embodiment these esters are obtained from a straight or branched amino alcohol comprising from 1 to 17 carbon atoms and from a straight or branched chain fatty acid comprising at least 12 carbon atoms, preferably from 14 to 22 carbon atoms. In one embodiment the preferred oils are mono- or diesters.

In one embodiment, the oil of the emulsion contains (at least 50%) of one or more hydrocarbon of oils comprising only carbon and hydrogen, which may be volatile or nonvolatile and may be mineral or synthetic. Suitable hydrocarbon oils include squalane, hydrogenated polyisobutene and hydrocarbon oils with a branched chain which preferably comprise from 6 to 20 and better still from 6 to 18 carbon atoms, for isohexadecane, isododecane, isoparaffins and mixtures thereof.

In one embodiment for certain applications, the oil includes gasoline, diesel fuel, kerosene or the like.

The oil is present in the range from about 99 wt.% to about 1 wt.%, in another embodiment in the range from about 95 wt.% to about 5 wt.%, and in another embodiment in the range from about 10 wt.% to about 30 wt.% of the internal phase of the water-in-oil emulsion.

One method for preparing the water-in-oil-in-water emulsion, involves adding the water-in-oil emulsion to an aqueous water phase which serves as the external phase of the multiple emulsions. In another embodiment, the external aqueous phase may be added to the water-in-oil emulsion.

The external aqueous phase may be any acceptable water based material and includes tap water, demineralized water, deionized water, floral water or the like. The water may be used alone or in combination. The external water phase can be the same or different than the water used in the aqueous phase of the water-in-oil emulsion.

The proportion of the aqueous phase in the internal water-in-oil emulsion to the external aqueous phase can be in the range of about 10 to 90:90 to 10, in another embodiment in the range of about 50 to 90:10 to 50 and in another embodiment in the range of about 20 to 40:60 to 80.

Added to the external aqueous phase, is a standard emulsifier having an HLB in the range of about 8 to about 20. The emulsifiers include ethoxylates, nonionic ethoxylated fatty acids, esters, sorbitan esters, alkylphenols sorbitan monolaurate and the like. Other useful emulsifiers include carboxylates including amine salts, metallic salts and the like, alkylarylsulfonates, amine oxides, poly(oxyalkylene) compounds, including block copolymers comprising alkylene oxide repeat units, carboxylated alcohol ethoxylates, ethoxylated alcohols, ethoxylated alkylphenols, ethoxylated amines and amides, ethoxylated fatty acids, ethoxylated fatty esters and oils, fatty esters, fatty acid amides, including but not limited to amides from tall oil fatty acids and polyamides, ethoxylated glycerol esters, ethoxylated glycol esters, ethoxylated sorbitan esters, imidazoline derivatives, lecithin and derivatives, lignin and derivatives, monoglycerides and derivatives, olefin sulfonates, phosphate esters and derivatives, propoxylated and ethoxylated fatty acids or alcohols or alkylphenols, sorbitan derivatives, sucrose esters and derivatives, sulfates or alcohols or ethoxylated alcohols or fatty esters, sulfonates of dodecyl and tridecyl benzenes or condensed naphthalenes or petroleum, sulfosuccinates and derivatives, and tridecyl and dodecyl benzene sulfonic acids.

However, any type of emulsifier meeting the HLB requirement can be used. Examples of other emulsifiers of this type can be found in McCutcheon's, Vol 1:Emulsifiers & Detergents, 2000.

In one embodiment the emulsifier is Tween 20 (POE(20) sorbitan monolaurate) with an HLB of about 16.7, and Chemonic S-20 (a 20 exthoxylate stearyl alcohol with an HLB of about 15.0).

The emulsifiers may be used alone or in combination. The emulsifier for the external aqueous phase is present in the range from about 0.01 wt.% to about 10 wt.%, in another embodiment the range from about 0.05 wt.% to about 7 wt.% and in another embodiment in the range from about 0.1 wt.% to about 5 wt.% of the multiple emulsion.

The additives, depending on their nature, may be introduced into the oil phase, or into either of the aqueous phases or combinations thereof. The possible additional additives and/or their quantities are chosen so that the advantageous properties of the multiple emulsions are not or not substantially adversely affected by the addition of the additives.

The multiple emulsion can be thickened by using a thickener in the external aqueous phase, the internal aqueous phase, the oil phase or combinations thereof. If a thickener is used in any of the phases it can be the same or different or mixture thereof.

Thickeners for use in either aqueous phase include gums, such as xanthan gum, cellulosics, chitosan, starches; silicates, magnesium aluminum silicates, hydroxyethylcellulose (such as the commercial product Natrosol cellulose or Cellosize), hydroxypropylcellulose (such as the commercial product Klucel), xanthan gums (such as the commercial product Rhodicare CFT from Rhodia) glucose-mannose polysaccharides, such as N-hance HP40 or N-hance HP40S, ammonium poly(acryldimethyltauramde-co-vinylformamide), also referred to as AMPS/VIFA copolymer, available commercially from Clariant Corporation, Charlotte, N.C. under the name trade name Aristoflex AVC; stearyl alcohol, cetyl alcohol, cetearyl alcohol, and various clays, such as hectorites, smectites and bentonites, including commercials products like Southern Clay Gel White MAS 100 .

Thickeners for use in either aqueous phase further include crosslinked polyacrylic acid copolymer such as Carbopol^{®} ETD 2020 available from Noveon, modified crosslinked polyacrylate polymer such as Carbopol^{®} Ultrez available from Noveon, polyethoxylates of-methyl glucose and their derivatives, such as Glucamate LT available from (Chemron), PEG-120 methyl glucose dioleate, such as Glucamate^{™} DOE 120 available from (Chemron), starch, modified potato starch such as Structure^{®} XL, Structure^{®} ZEA available from National Starch and the like.

Thickeners for use in the oil phase include ethylene/ propylene/styrene copolymers, butylene/ethylene/styrene copolymers; commercial products like Versagel M750, Versagel ME 750, Versagel MP 500, Versagel MD 1600, available from Penreco; Transgel 105 and Transgel 110 available from Rita; polyisobutylene, hydrogenated polyisobutylene, waxes, such as polyethylene wax, beeswax and the like: oil-soluble polyacrylates, oil soluble polymethacrylates, olefin polymers, olefin co-polymers, functionialized olefin copolymers, olefin terpolymers and functionalized olefin terpolymers, hydrophobically modified clays, silicas, and copolymers of styrene and olefins.

The thickener may be used alone or in combination. The amount of thickener used will depend on the desired viscosity and/or desired stability of the multiple emulsion and is in the range from about 0 wt. % to about 10 wt.%, in another embodiment the range from about 0.1 wt.% of about 9 wt.%, and in another embodiment the range from about 1 wt.% from about 5 wt.% of the multiple emulsion.

The external aqueous phase, the aqueous phase in the internal emulsion or both phases may include water soluble additives, water dispersible additives and mixtures thereof. The water soluble additives may be the same or different or combinations thereof in either the external or internal or both of the aqueous phases of the multiple emulsion. The water soluble additives may be used alone or in combination. The water dispersible additives may be the same or different or combinations thereof in either the external or internal or both of the aqueous phases of the multiple emulsion. The water dispersible additives may be used alone or in combination.

Water soluble additives include solvents such as propylene glycol, active agents, preservatives, antioxidants, complexing agents, perfumes, fillers, bactericides, odor absorbers, color matter, dyes, lipid vesicles and the like. Moisturizers such as protein hydrolysates and polyols such as glycerin; glycols such as polyethylene glycols; sugar derivatives; natural extracts; skin lightening agents, bleaching agents, botanicals, refatting agents, skin and hair conditioners; vitamins, urea; caffeine; depigmenting agents such as kojic acid and caffeic acid; beta-hydroxy acids such as salicylic acid and its derivatives; alpha-hydroxy acids such as lactic acid and glycolic acid; emollients and humectants, such as ethoxylated methyl glucosides and acylated ethoxylated methyl glucosides; dihydroxyacetone, amino acids and mixture of amino acids, inorganic salts, inorganic oxides, sunscreens, retinoids such as retinol and its derivatives and carotenoids; organic and inorganic screening agents; hydrocortisone; DHEA; melatonin; algal, fungal, plant, yeast or bacterial extracts; proteins, hydrolysed, partially hydrolysed or unhydrolyzed; enzymes and mixtures thereof.

The water soluble additives can also include fertilizers, such as ammonium nitrate, urea, insecticides, fungicides, and bacteriocides.

The water soluble additives are in the range from about 0 % to about 30 %, and in another embodiment about 0.01 % to about 20 %, and in another embodiment from 0.1 % to 5% and in another embodiment from .5 % to 3 % of the total weight of the multiple emulsions.

The water dispersible additives include clays, pigments, aluminum oxides, silicates, talc, magnesium silicates, titanium dioxide, and zinc oxide.

The water dispersible additives are in the range from about 0 % to about 30 %, in another embodiment about 0.01 % to about 20 % and in another embodiment about 0.5 % to about 10 % of the total weight of the multiple emulsions.

The oil phase includes any water immiscible additives acceptable in oil, such an oil being defined for the present purpose as any acceptable material which is substantially insoluble in water. As the oils can perform different functions in the composition, the specific choice is dependent on the purpose for which it is intended.

The oil phase includes lipophilic additives, fatty acids, fatty alcohols, gums, waxes, silicone gums, oily gelling agents, organic particles, inorganic particles and the like. The oil phase additives may be used alone or in combination.

The oil phase additives include the oil thickeners listed above; vitamins; organic sunblocks, such as avobenzone, octocrylene, cinnamate esters, salicylate esters; refatting agents; skin and hair conditioners; emollients and moisturizers; lanolins and any other additives that add to the performance of the emulsion in the intended end-use application.

The oil phase additives are in the range from about 0 wt.% to about 30 wt.%, in another embodiment in the range from about 0.1 wt.% to about 10 wt.%, in another embodiment in the range from about 1 wt.% to about 5 wt.% of the internal phase of the water-in-oil emulsion.

The multiple emulsions are conveniently but not necessarily prepared as conventional multiple emulsions are prepared. Initially, a water-in-oil emulsion is prepared according to standard procedure. Generally the water soluble ingredients are combined together in an aqueous vehicle, the oil soluble ingredients are combined in the oil vehicle, and the two phases are combined with the PIB and/or PIBSA emulsifier to form the water-in-oil emulsion. The water, the oil, the PIB and/or PIBSA emulsifier and desired additives are combined with stirring, shearing and combinations thereof, at a temperature in the range from ambient temperature to about 80°C. The water-in-oil emulsion is then incorporated into the external aqueous phase with emulsifiers and desired additives with mixing at a temperature in the range from about ambient temperature to about 65°C. The resulting emulsion is stable and intact as a multiple emulsion.

The water phase of the internal water-in-oil emulsion is comprised of droplets having a mean diameter of about 0.1 to about 5 microns, in one embodiment about 0.5 to about 3 microns or less, in another embodiment about 2 microns or less, in another embodiment about 1 micron or less. The internal water-in-oil emulsion in the external aqueous phase is comprised of droplets of the internal water-in-oil emulsion having a mean diameter of about 1 to about 50 microns, in another embodiment about 30 microns or less, in another embodiment about 10 microns or less, in another embodiment about 5 microns or more, in another embodiment about 10 micron to about 5 microns.

The multiple emulsions of the present invention provide a number of advantages over traditional multiple emulsions. The system permits for a greater concentration of the internal emulsion in the multiple emulsion, thereby permitting a broader variety of textures, and a broader appeal to a wide range of products. The multiple emulsions can be used to deliver a number of different types of active materials, partitioned among the various phases of the final product. This can be particularly important in a system in which there are several actives that may not be compatible together, or that may not exhibit optimum activity in the same environment. The multiple emulsion is stable.

The emulsions can essentially be used for any type of application in which an emulsion is used in particular for industrial, household and consumer products. Applications areas include metal working, horticulture, agriculture, coatings/ paints/ inks, lubricants and fuels. The multiple emulsion of this invention finds particular application in a large number of personal care applications, such as the treatment of, the protection of, and the care of skin, lip and nail, make-up removal and/or for cleansing the skin, and personal care products, such as sunscreens/self-tanners, rinse-off hair conditioners, liquid make ups and the like.

The invention will be further illustrated by the following non-limiting examples.

### Specific Embodiment

**Example 1:** The oil phase of the primary emulsion includes white oil (about 15.7 g) as the oil, and Chemccinate 2000 (about 6.3g) [low color PIBSA/triethanolamine (1:1)m from Chemron] as the emulsifier. Dihydroxyacetone (DHA) (about 10.0g) is the tanning active ingredient and it is combined with about 66.95 g of water and 1.05 g of sodium chloride to prepare the aqueous phase of the primary emulsion. Both phases are heated to about 70°C, and the aqueous phase is added dropwise to the oil phase over about 20 minutes while mixing with a Heidolph mixer at about 600 rpm. Once the addition is complete, the water-in-oil emulsion is homogenized at about 7000 rpm for about 10 minutes. The resulting emulsion is the internal water-in-oil emulsion of the multiple emulsion. The droplet size is in the about 0.5-2 micron range for the primary water-in-oil emulsion.

The external aqueous phase is prepared by combining the following ingredients at 50C with stirring: water (about 26.05g), preservative (about 0.2g), sodium chloride (about 0.28g), Chemonic S-20 (stearyl alcohol 20 ethoxylate) (about 3.0g), smectite clay (about 1.33g), and an amino acid complex (about 1.0g). The internal water-in-oil emulsion (about 66.8g) is then added to the external aqueous phase over about 20 minutes at about 500 rpm. To this multiple emulsion is added a mixture of xanthan gum (about 0.67g) and 2-ethylhexyl stearate (about 0.67g) and the mixture is stirred for an additional about 30 minutes, followed by addition of a preservative.

This emulsion has shown excellent stability after 5 freeze thaw cycles (about -18C to ambient temperature) and about 43°C storage (two weeks) based on visual inspection, microscopy, and viscosity.

**Example 2:** The oil phase of the primary emulsion includes octyl stearate (about 15.7 g) as the oil, Chemccinate 2000 (about 6.3g) [low color PIBSA/triethanolamine (1:1)m from Chemron] as the emulsifier. Dihydroxyacetone (DHA) (about 10.0g) is the tanning active ingredient and it is combined with about 66.95 g of water and about 1.05 g of sodium chloride to prepare the aqueous phase of the primary emulsion. Both phases are heated to about 70°C, and the aqueous phase is added dropwise to the oil phase over about 20 minutes while mixing with a Heidolph mixer at about 600 rpm. Once the addition is complete, the water-in-oil emulsion is homogenized at about 7000 rpm for about 10 minutes. The resulting emulsion is the internal water-in-oil emulsion of the multiple emulsion. The droplet size is in the about 0.5-2 micron range for the primary water-in-oil emulsion.

The external aqueous phase is prepared by combining the following ingredients at about 50°C with stirring: water (about 26.05g), preservative (about 0.2g), sodium chloride (about 0.28g), Chemonic S-20 (stearyl alcohol 20 ethoxylate) (about 3.0g), smectite clay (about 1.33g), and an amino acid complex (about 1.0g). The internal water-in-oil emulsion (about 66.8g) is then added to the external aqueous phase over about 20 minutes at about 500 rpm. To this multiple emulsion is added a mixture of xanthan gum (about 0.67g) and 2-ethylhexyl stearate (about 0.67g) and the mixture is stirred for an additional about 30 minutes, followed by addition of a preservative.

This emulsion has shown very good stability after 5 freeze thaw cycles (about -18°C to ambient temperature) and about 43°C storage (two weeks) based on visual inspection, microscopy, and viscosity.

**Example 3:** The oil phase of the primary emulsion is prepared by combining white oil (about 14.5 g), petrolatum (about 0.5g), Chemccinate 2000 (about 7.5g) [low color PIBSA/triethanolamine (1:1)m from Chemron] and sorbitan monoisostearate (about 1.5g). The aqueous phase is prepared by combining dihydroxyacetone (DHA) (about 10.0g), about 63.0 g of water and about 2.0 g of a smectite clay, about 0.5g of xanthan gum and about 0.5g of white oil. Both phases are heated to about 70°C, and the aqueous phase is added dropwise to the oil phase over about 20 minutes while mixing with a Heidolph mixer at about 600 rpm. Once the addition is complete, the water-in-oil emulsion is homogenized at about 7000 rpm for about 10 minutes. The resulting emulsion is the internal water-in-oil emulsion of the multiple emulsion. The droplet size is in the about 0.5-2 micron range for the primary water-in-oil emulsion.

The external aqueous phase is prepared by combining the following ingredients at about 50°C with stirring: water (about 34.2g), Chemonic S-20 (stearyl alcohol 20 ethoxylate) (about 3.0g), smectite clay (about 1.4g), and an amino acid complex (about 1.0g). The internal water-in-oil emulsion (about 60.0g) is then added to the external aqueous phase over about 20 minutes at about 500 rpm. To this multiple emulsion is added a mixture of xanthan gum (about 0.1g) and white oil (about 0.1g) and the mixture is stirred for an about additional about 30 minutes, followed by addition of a preservative (about 0.2g).

This emulsion has shown excellent stability after 5 freeze thaw cycles (about -18°C to ambient temperature) and about 43°C storage (two weeks) based on visual inspection, microscopy, and viscosity.

**Example 4:** The oil phase of the primary emulsion is prepared by combining white oil (about 15.7 g), Chemccinate 2000 (about 6.3g) [low color PIBSA/triethanolamine (1:1)m from Chemron]. The aqueous phase is prepared by combining dihydroxyacetone (DHA) (about 10.0g), sodium chloride (about 1.0g), about 65.0 g of water and about 2.0 g of a smectite clay. Both phases are heated to about 70°C, and the aqueous phase is, added dropwise to the oil phase over about 20 minutes while mixing with a Heidolph mixer at about 600 rpm. Once the addition is complete, the water-in-oil emulsion is homogenized at about 7000 rpm for about 10 minutes. The resulting emulsion is the internal water-in-oil emulsion of the multiple emulsion. The droplet size is in the about 0.5-2 micron range for the primary water-in-oil emulsion.

The external aqueous phase is prepared by combining the following ingredients at about 50°C with stirring: water (about 25.12g), sodium chloride (about 0.28g), Chemonic S-20 (stearyl alcohol 20 ethoxylate) (about 3.0g), smectite clay about 2.8g), and an amino acid complex (about 1.0g). The internal water-in-oil emulsion (about 66.6g) is then added to the external aqueous phase over about 20 minutes at about 500 rpm. To this multiple emulsion is added a mixture of xanthan gum (about 0.5g) and white oil (about 0.5g) and the mixture is stirred for an additional about 30 minutes, followed by addition of a preservative (about 0.2g).

This emulsion has shown excellent stability after 5 freeze thaw cycles (about -18°C to ambient temperature) and about 43°C storage (two weeks) based on visual inspection, microscopy, and viscosity.

## Claims

1. A multiple water-in-oil-in-water emulsion composition comprising:
(1) a discontinuous phase, wherein that discontinuous phase comprises a) a water discontinuous phase, b) an oil continuous phase, c) an emulsifier selected from the group consisting of a polyisobutenyl succinic anhydride-derived emulsifier(s), a polyisobutene derived emulsifier(s) and mixtures thereof, d) optionally a co-emulsifier and e) optionally thickeners for the discontinuous phase resulting in an internal water-in-oil emulsion; and
(2) a continuous phase, wherein that continuous phase is an external aqueous phase comprising a) water b) the internal water-in-oil emulsion, c) an oil-in-water emulsifier and d) optionally thickeners for the external aqueous phase; and
wherein the emulsifier is used in the range of 0.1 wt.% to 30 wt.% of the final multiple emulsion; and
wherein the water phase of the internal water-in-oil emulsion is comprised of droplets having a mean diameter of 0.1 to 5 µm;
resulting in a stable water-in-oil-in-water multiple emulsion.

2. The composition of claim 1 wherein the polyisobutenyl succinic anhydride-derived emulsifier comprises the reaction product of poly(isobutylene) with maleic anhydride, poly(isobutylene) succinic anhydride, poly(isobutylene) succinic anhydride derivatives, functionalized poly(isobutylene) succinic anhydride, the reaction product of poly(isobutylene) succinic anhydride with alcohol, amines, polyols, polyamines, and alkanolamines, the reaction product of poly(isobutylene) succinic anhydride with triethanolamine, the reaction product of poly(isobutylene) succinic anhydride with glycerol, the reaction product of poly(isobutylene) succinic anhydride with sodium hydroxide, or combinations thereof.

3. The composition of claim 1 wherein the polyisobutylene derived emulsifier is selected from the group consisting of at least one of the following: polyisobutenyl substituted phenols, amino polyisobutenyl phenols, polyisobutenyl amine and mixtures thereof; the reaction products of polyisobutylene with α,β-unsaturated olefins followed by further functionalized by reaction with nucleophiles like water, alcohol, polyols, amines, polyamines, alkanolamines, and inorganic bases; the reaction products of polyisobutylene with glyoxylic acid, lower alkyl glyoxylates, such as methyl glyoxylate, or lower alkyl hemiacetals, such acid methyl glyoxylate methyl hemiacetal, followed by further functionalized with primary or secondary amines, primary or secondary alkanolamines, or polyamines.

4. The composition of claim 1 wherein the coemulsifier comprises sorbitan monooleate, sorbitan monoisostearate, glycerol monooleate, oleyl alcohol 2-ethoxylate, lecithin, sorbitan monolaurate, sorbitan monopalmitate, sorbitan monostearate, sorbitan sesquistearate, sorbitan trioleate, stearyl alcohol 2-ethoxylate, glycerol monostearate, sorbitan dioleate, wool fat, methyl glucoside dioleate, polyglyceryl-3 diisostearate, polyethylene glycol 200 distearate, methyl glucose sesquistearate, polyethylene glycol 200 monostearate, alcohol alkoxylates, copolymers of various alkoxylates, alkyl amines or alcohols or amides, ethoxylated alkyl amines or amides, betaines, compatible sulfonates or sulfates or sulfosuccinates or phosphonates or borates or amine salts or carboxylates, or mixtures thereof and the coemulsifier is present in the range from 0 wt.% to 10 wt.% of the internal water-in-oil emulsion.

5. The composition of claim 1 wherein the aqueous material comprises tap water, demineralized water, deionized water, floral water or combinations thereof; and
wherein the oil comprises fatty substances, volatile oils, non-volatile oils or mixtures thereof; and
wherein the external aqueous phase comprises tap water, demineralized water, deionized water, floral water or combinations thereof;
and the water is present in the range of 1 wt.% to 99 wt.% of the internal phase of the water emulsion; and
the oil is present in the range of 99 wt.% to 1 wt.% of the water-in-oil emulsion; and
wherein the internal water-in-oil emulsion to the external aqueous phase is in the range of 10 to 90:90 to 10.

6. The composition of claim 1 wherein the external aqueous phase emulsifier comprises ethoxylates, nonionic ethoxylated fatty acids, esters, sorbitan esters, alkylphenols, sorbitan monolaurate, carboxylates, amine salts, metallic salts, alkylarylsulfonates, amine oxides, poly(oxyalkylene) compounds, including block copolymers comprising alkylene oxide repeat units, carboxylated alcohol ethoxylates, ethoxylated alcohols, ethoxylated alkylphenols, ethoxylated amines and amides, ethoxylated fatty acids, ethoxylated fatty esters and oils, fatty esters, fatty acid amides, including but not limited to amides from tall oil fatty acids and polyamides, ethoxylated glycerol esters, ethoxylated glycol esters, ethoxylated sorbitan esters, imidazoline derivatives, lecithin and derivatives, lignin and derivatives, monoglycerides and derivatives, olefin sulfonates, phosphate esters and derivatives, propoxylated and ethoxylated fatty acids or alcohols or alkylphenols, sorbitan derivatives, sucrose esters and derivatives, sulfates or alcohols or ethoxylated alcohols or fatty esters, sulfonates of dodecyl and tridecyl benzenes or condensed naphthalenes or petroleum, sulfosuccinates and derivatives, and tridecyl and dodecyl benzene sulfonic acids or mixtures thereof and is present in the range from 0.01 wt.% to 10 wt.% of the multiple emulsion.

7. The composition of claim 1 wherein the multiple emulsion is thickened by a thickener comprising xanthan gum, cellulosics, chitosan, starches, silicates, magnesium aluminum silicates, hydroxyethylcellulose, xanthan gums, glucose-mannose polysaccharides, ammonium poly(acryldimethyltauramide-co-vinylformamide), stearyl alcohol, cetyl alcohol, cetearyl alcohol, clays, hectorites, smectites, bentonites, crosslinked polyacrylic acid copolymer, modified crosslinked polyacrylate polymer, polyethoxylates of methyl glucose and their derivatives, PEG-120 methyl glucose dioleate, starch, modified potato starch, ethylene/propylene/styrene copolymers, butylenes/ethylene/styrene copolymers, polyisobutylene, hydrogenated polyisobutylene, waxes, polyethylene wax, beeswax, oil soluble polyacrylates, oil soluble polymethacrylates, olefin polymers, olefin co-polymers, functionalized olefin copolymers, olefin terpolymers, functionalized olefin terpolymers, hydrophobically modified clays, silicas, and copolymers of styrene and olefins or the mixtures thereof and wherein the thickener is in the range from 0 wt.% to 10 wt.% of the multiple emulsion.

8. The composition of claim 1 wherein the composition comprises water soluble additives comprising propylene glycol, active agents, preservatives, antioxidants, complexing agents, perfumes, fillers, bactericides, odor absorbers, color matter, dyes; lipid vesicles, protein hydrolysates and polyols such as glycerin; glycols such as polyethylene glycols; sugar derivatives; natural extracts; skin lightening agents, bleaching agents, botanicals, refatting agents, skin and hair conditioners; vitamins, urea; caffeine; depigmenting agents such as kojic acid and caffeic acid; beta-hydroxy acids such as salicylic acid and its derivatives; alpha-hydroxy acids such as lactic acid and glycolic acid; emollients and humectants, such as ethoxylated methyl glucosides and acylated ethoxylated methyl glucosides; dihydroxyacetone, amino acids and mixture of amino acids, inorganic salts, inorganic oxides, sunscreens, retinoids such as retinol and its derivatives and carotenoids; organic and inorganic screening agents; hydrocortisone; DHEA; melatonin; algal, fungal, plant, yeast or bacterial extracts; proteins, hydrolysed, partially hydrolysed or unhydrolyzed; enzymes or mixtures thereof and wherein the water soluble additives are in the range from 0 wt.% to 30 wt.% of the multiple emulsion.

9. The composition of claim 1 wherein the composition comprises water dispersable additives comprising clays, pigments, aluminum oxides, silicates, talc, magnesium silicates, titanium dioxide, zinc oxide or mixtures thereof and wherein the water dispersable additives are in the range from 0 wt.% to 30 wt.% of the multiple emulsion.

10. The composition of claim 1 wherein the oil composition comprises oil phase additives comprising lipophilic additives, fatty acids, fatty alcohols, gums, waxes, silicone gums, oil gelling agents, organic particles, inorganic particles, thickeners, vitamins, organic sunblocks, avobenzone, octocrylene, cinnamate esters, salicylate esters, refatting agents, skin conditioners, hair conditioners, emollients, moisturizers, lanolins or mixtures thereof and wherein the oil phase additives are in the range from 0 wt.% to 30 wt.% of the internal phase of the water-in-oil emulsion.

11. The composition of claim 1 wherein the internal water-in-oil emulsion in the external aqueous phase is comprised of droplets of the internal water-in-oil emulsion having a mean diameter of 1 to 50 µm.

12. The composition of claim 1 wherein the oil-in-water emulsifier has an HLB of 8 to 20.

13. The composition of claim 1 used as multiple emulsion for products selected from the group consisting of industrial products, household products, consumer products, personal care products, metal working products, horticulture products, agriculture products, coating products, paint products, ink products, lubricant products, fuel products and combinations thereof.

14. A method of making a multiple emulsion composition comprising combining an aqueous component in the range of 1 wt.% to 99 wt.% and an oil phase in the range of 99 wt.% to 1 wt.% with an emulsifier selected from the group consisting of poly(isobutenyl) succinic anhydride-derived emulsifier, a polyisobutylene derived emulsifier and mixtures thereof to produce an internal water-in-oil emulsion, and then combining the internal water-in-oil in an external aqueous phase at a ratio of 10 to 90:90 to 10 with an emulsifier with a HLB of 8 to 20 resulting in a stable multiple emulsion.

## Patentansprüche

1. Mehrfach-Wasser-in-Öl-in-Wasser-Emulsionszusammensetzung, umfassend:
(1) eine diskontinuierliche Phase, wobei diese diskontinuierliche Phase Folgendes umfasst: a) eine diskontinuierliche Wasserphase, b) eine kontinuierliche Ölphase, c) einen Emulgator, ausgewählt aus der Gruppe bestehend aus (einem) von Polyisobutenylbernsteinsäureanhydrid stammenden Emulgator(en), (einem) von Polyisobuten stammenden Emulgator(en) und Mischungen davon, d) gegebenenfalls einem Coemulgator und e) gegebenenfalls Verdickungsmitteln für die diskontinuierliche Phase, die in eine interne Wasser-in-Öl-Emulsion resultieren, und
(2) eine kontinuierliche Phase, wobei diese kontinuierliche Phase eine externe wässrige Phase ist, die Folgendes umfasst: a) Wasser, b) die interne Wasser-in-Öl-Emulsion, c) einen Öl-in-Wasser-Emulgator und d) gegebenenfalls Verdickungsmittel für die externe wässrige Phase und
wobei der Emulgator im Bereich von 0,1 Gew.-% bis 30 Gew.-% der fertigen Mehrfachemulsion verwendet wird, und
wobei die Wasserphase der internen Wasser-in-Öl-Emulsion aus Tröpfchen mit einem mittleren Durchmesser von 0,1 bis 5 µm besteht,
was in einer stabilen Wasser-in-Öl-in-Wasser-Emulsion resultiert.

2. Zusammensetzung nach Anspruch 1, wobei der von Polyisobutenylbernsteinsäureanhydrid stammende Emulgator das Produkt der Reaktion von Poly(isobutylen) mit Maleinsäureanhydrid, Poly(isobutylen)bernsteinsäureanhydrid, Poly(isobutylen)bernsteinsäureanhydrid-Derivaten, funktionalisiertem Poly(isobutylen)bernsteinsäureanhydrid, das Produkt der Reaktion von Poly(isobutylen)bernsteinsäureanhydrid mit einem Alkohol, Aminen, Polyolen, Polyaminen und Alkanolaminen, das Produkt der Reaktion von Poly(isobutylen)bernsteinsäureanhydrid mit Triethanolamin, das Produkt der Reaktion von Poly(isobutylen)bernsteinsäureanhydrid mit Natriumhydroxid oder Kombinationen davon umfasst.

3. Zusammensetzung nach Anspruch 1, wobei der von Polyisobutylen stammende Emulgator ausgewählt ist aus der Gruppe, die aus wenigstens Folgendem besteht: polyisobutenylsubstituierten Phenolen, Aminopolyisobutenylphenolen, Polyisobutenylamin und Mischungen davon, den Produkten der Reaktion von Polyisobutylen mit α,β-ungesättigten Olefinen, gefolgt von einer weiteren Funktionalisierung durch eine Reaktion mit Nucleophilen wie Wasser, Alkohol, Polyolen, Aminen, Polyaminen, Alkanolaminen und anorganischen Basen, den Produkten der Reaktion von Polyisobutylen mit Glyoxylsäure, Niederalkylglyoxylaten wie Methylglyoxylat, gefolgt von einer weiteren Funktionalisierung mit primären oder sekundären Aminen, primären öder sekundären Alkanolaminen oder Polyaminen.

4. Zusammensetzung nach Anspruch 1, wobei der Coemulgator Sorbitanmonooleat, Sorbitanmonoisostearat, Glycerinmonooleat, Oleylalkohol-2-ethoxylat, Lecithin, Sorbitanmonolaurat, Sorbitanmonopalmitat, Sorbitanmonostearat, Sorbitansesquistearat, Sorbitantrioleat, Stearylalkohol-2-ethoxylat, Glycerinmonostearat, Sorbitandioleat, Wollfett, Methylglucosiddioleat, Polyglyceryl-3-diisostearat, Polyethylenglycol-200-distearat, Methylglucosesesquistearat, Polyethylenglycol-200-monostearat, Alkoholalkoxylate, Copolymere von verschiedenen Alkoxylaten, Alkylaminen oder Alkoholen oder Amiden, ethoxylierten Alkylaminen oder Amiden, Betainen, verträgliche Sulfonate oder Sulfate oder Sulfosuccinate oder Phosphonate oder Borate oder Aminsalze oder Carboxylate oder Mischungen davon umfasst und wobei der Coemulgator im Bereich von 0 Gew.-% bis 10 Gew.-% der internen Wasser-in-Öl-Emulsion vorhanden ist.

5. Zusammensetzung nach Anspruch 1, wobei das wässrige Material Leitungswasser, demineralisiertes Wasser, deionisiertes Wasser, Blumenwasser oder Kombinationen davon umfasst, und
wobei das Öl Fettsubstanzen, flüchtige Öle, nichtflüchtige Öle oder Mischungen davon umfasst und
wobei die externe wässrige Phase Leitungswasser, demineralisiertes Wasser, deionisiertes Wasser, Blumenwasser oder Kombinationen davon umfasst
und das Wasser im Bereich von 1 Gew.-% bis 99 Gew.-% der internen Phase der Wasseremulsion vorhanden ist und
das Öl im Bereich von 99 Gew.-% bis 1 Gew.-% der Wasser-in-Öl-Emulsion vorhanden ist und
wobei das Verhältnis der internen Wasser-in-Öl-Emulsion zur externen wässrigen Phase im Bereich von 10 bis 90:90 bis 10 beträgt.

6. Zusammensetzung nach Anspruch 1, wobei der Emulgator der externen wässrigen Phase Ethoxylate, nichtionische ethoxylierte Fettsäuren, Ester, Sorbitanester, Alkylphenol, Sorbitanmonolaurat, Carboxylate, Aminsalze, Metallsalze, Alkylarylsulfonate, Aminoxide, Poly(oxyalkylen)-Verbindungen einschließlich Block-Copolymeren, die Alkylenoxid-Repetiereinheiten umfassen, carboxylierte Alkoholethoxylate, ethoxylierte Alkohole, ethoxylierte Alkylphenole, ethoxylierte Amine und Amide, ethoxylierte Fettsäuren, ethoxylierte Fettester und -öle, Fettester, Fettsäureamide einschließlich, ohne darauf beschränkt zu sein, Amiden von Tallölfettsäuren und Polyamiden, ethoxylierte Glycerinester, ethoxylierte Glycolester, ethoxylierte Sorbitanester, Imidazolin-Derivate, Lecithin und Lecithinderivate, Lignin und Ligninderivate, Monoglyceride und Monoglyceridderivate, Olefinsulfonate, Phosphatester und Phosphatesterderivate, propoxylierte und ethoxylierte Fettsäuren oder Alkohole oder Alkylphenole, Sorbitanderivate, Saccharoseester und Saccharoseesterderivate, Sulfate oder Alkohole oder ethoxylierte Alkohole oder Fettester, Sulfonate von Dodecyl- und Tridecylbenzolen oder kondensierten Naphthalinen oder Petroleum, Sulfosuccinate und Sulfosuccinatderivate und Tridecyl- und Dodecylbenzolsulfonsäuren oder Mischungen davon umfasst und im Bereich von 0,01 Gew.-% bis 10 Gew.-% der Mehrfachemulsion vorhanden ist.

7. Zusammensetzung nach Anspruch 1, wobei die Mehrfachemulsion mit einem Verdickungsmittel verdickt ist, das Xanthangummi, Cellulosen, Chitosan, Stärken, Silicate, Magnesiumaluminiumsilicate, Hydroxyethylcellulose, Xanthangummen, Glucose-Mannose-Polysaccharide, Ammoniumpoly(acryldimethyltauramid-co-vinylformamid), Stearylalkohol, Cetylalkohol, Cetearylalkohol, Tone, Hectorite, Smectite, Bentonite, vernetztes Polyacrylsäure-Copolymer, modifiziertes vernetztes Polyacrylat-Polymer, Polyethoxylate von Methylglucose und Derivate davon, PEG-120-Methylglucosedioleat, Stärke, modifizierte Kartoffelstärke, Ethylen/Propylen/Styrol-Copolymere, Butylene/Ethylen/Styrol-Copolymere, Polyisobutylen, hydriertes Polyisobutylen, Wachse, Polyethylenwachs, Bienenwachs, öllösliche Polyacrylate, öllösliche Polymethacrylate, Olefinpolymere, Olefin-Copolymere, funktionalisierte Olefin-Copolymere, Olefin-Terpolymere, funktionalisierte Olefin-Terpolymere, hydrophob modifizierte Tone, Siiiciumdioxide und Copolymere von Styrol und Olefinen oder die Mischungen davon umfasst und wobei das Verdickungsmittel im Bereich von 0 Gew.-% bis 10 Gew.-% der Mehrfachemulsion vorliegt.

8. Zusammensetzung nach Anspruch 1, wobei die Zusammensetzung wasserlösliche Additive umfasst, die Propylenglycol, Wirkstoffe, Konservierungsmittel, Antioxidantien, Komplexierungsmittel, Parfüms, Füllstoffe, Bakterizide, geruchsabsorbierende Mittel, Farbstoffe, Lipidvesikel, Proteinhydrolysate und Polyole wie Glycerin, Glycole wie Polyethylenglycole, Zuckerderivate, natürliche Extrakte, Hautaufhellungsmittel, Bleichmittel, pflanzliche Mittel, Rückfettungsmittel, Haut- und Haarkonditionierungsmittel, Vitamine, Harnstoff, Koffein, Depigmentierungsmittel wie Kojisäure und Kaffeesäure, β-Hydroxysäuren wie Salicylsäure und Derivate davon, α-Hydroxysäuren wie Milchsäure und Glycolsäure, Weichmacher und Feuchthaltemittel wie ethoxylierte Methylglucoside und acylierte ethoxylierte Methylglycoside, Dihydroxyaceton, Aminosäuren und eine Mischung von Aminosäuren, anorganische Salze, anorganische Oxide, Sonnenschutzfilter, Retinode wie Retinol und Derivate davon und Carotenoide, organische und anorganische strahlungsabschirmende Mittel, Hydrocortison, DHEA, Melatonin, Algen-, Fungus-, Pflanzen-, Hefe- oder Bakterienextrakte, Proteine, hydrolysiert, teilweise hydrolysiert oder unhydrolysiert, Enzyme oder Mischungen davon umfasst und wobei die wasserlöslichen Additive im Bereich von 0 Gew.-% bis 30 Gew.-% der Mehrfachemulsion vorliegen.

9. Zusammensetzung nach Anspruch 1, wobei die Zusammensetzung wasserdispergierbare Additive umfasst, die Tone, Pigmente, Aluminiumoxide, Silicate, Talk, Magnesiumsilicate, Titandioxid, Zinkoxid oder Mischungen davon umfasst und wobei die wasserdispergierbaren Additive im Bereich von 0 Gew.-% bis 30 Gew.-% der Mehrfachemulsion vorliegen.

10. Zusammensetzung nach Anspruch 1, wobei die Ölzusammensetzung Ölphasenadditive umfasst, die lipophile Additive, Fettsäuren, Fettalkohole, Gummen, Wachse, Silicongummis, Ölgeliermittel, organische Teilchen, anorganische Teilchen, Verdickungsmittel, Vitamine, organische Sun-Blocker, Avobenzon, Octocrylen, Cinnamatester, Salicylatester, Rückfettungsmittel, Hautkonditionierungsmittel, Haarkonditionierungsmittel, Weichmacher, Moisturizer, Lanoline oder Mischungen davon umfassen und wobei die Ölphasenadditive im Bereich von 0 Gew.-% bis 30 Gew.-% der internen Phase der Wasser-in-Öl-Emulsion vorliegen.

11. Zusammensetzung nach Anspruch 1, wobei die interne Wasser-in-Öl-Emulsion in der externen wässrigen Phase aus Tröpfchen der internen Wasser-in-Öl-Emulsion mit einem mittleren Durchmesser von 1 bis 50 µm besteht.

12. Zusammensetzung nach Anspruch 1, wobei das ÖI-in-Wasser-Emulgiermittel ein HLB von 8 bis 20 hat.

13. Zusammensetzung nach Anspruch 1, die als Mehrfachemulsion für Produkte verwendet wird, die ausgewählt sind aus der Gruppe bestehend aus industriellen Produkten, Haushaltsprodukten, Verbraucherprodukten, Körperpflegeprodukten, Metallbearbeitungsprodukten, Gartenbauprodukten, Anstrichmittelprodukten, Tintenprodukten, Schmiermittelprodukten, Kraftstoffprodukten und Kombinationen davon.

14. Verfahren zur Herstellung einer Mehrfach-Emulsionszusammensetzung, umfassend das Vereinigen einer wässrigen Komponente im Bereich von 1 Gew.-% bis 99 Gew.-% und einer Ölphase im Bereich von 99 Gew.-% bis 1 Gew.-% mit einem Emulgator, der ausgewählt ist aus der Gruppe bestehend aus einem von Poly(isobutylenyl)bernsteinsäureanhydrid stammenden Emulgator, einem von Polyisobutylen stammenden Emulgator und Mischungen davon, zur Herstellung einer internen Wasser-in-Öl-Emulsion und dann das Vereinigen der internen Wasser-in-Öl-Emulsion mit einer externen wässrigen Phase mit einem Verhältnis von 10 bis 90:90 bis 10 mit einem Emulgator mit einem HLB von 8 bis 20, was in einer stabilen Mehrfachemulsion resultiert.

## Revendications

1. Composition d'émulsion multiple eau-huile-eau comprenant :
(1) une phase discontinue, dans laquelle la phase discontinue comprend a) une phase aqueuse discontinue, b) une phase continue huileuse, c) un émulsifiant choisi dans le groupe consistant en un/des émulsifiant(s) dérivé(s) d'anhydride polyisobutényle succinique, un/des émulsifiant(s) dérivé(s) de polyisobutène et leurs mélanges, d) facultativement un co-émulsifiant et e) facultativement des épaississants pour la phase discontinue, ce qui résulte en une émulsion interne eau-huile ; et
(2) une phase continue, dans laquelle ladite phase continue est une phase aqueuse externe comprenant a) de l'eau, b) l'émulsion interne eau-huile, c) un émulsifiant huile-eau et d) facultativement des épaississants pour la phase aqueuse externe ; et dans laquelle l'émulsifiant est utilisé dans une plage de 0,1 % en poids à 30 % en poids de l'émulsion multiple finale ; et
dans laquelle la phase aqueuse de l'émulsion eau-huile interne est composée de gouttelettes ayant un diamètre moyen de 0,1 à 5 µm ;
ce qui résulte en une émulsion multiple eau-huile-eau stable.

2. Composition selon la revendication 1, dans laquelle l'émulsifiant dérivé d'anhydride polyisobutényle succinique comprend le produit de réaction du poly(isobutylène) avec l'anhydride maléique, l'anhydride poly(isobutylène) succinique, les dérivés d'anhydride poly(isobutylène) succinique, l'anhydride poly(isobutylène) succinique fonctionnalisé, le produit de réaction de l'anhydride poly(isobutylène) succinique avec de l'alcool, des amines, des polyols, des polyamines, et des alkanolamines, le produit de réaction de l'anhydride poly(isobutylène) succinique avec la triéthanolamine, le produit de réaction de l'anhydride poly(isobutylène) succinique avec le glycérol, le produit de réaction de l'anhydride poly(isobutylène) succinique avec l'hydroxyde de sodium, ou leurs combinaisons.

3. Composition selon la revendication 1, dans laquelle l'émulsifiant dérivé de polyisobutylène est choisi dans le groupe consistant en au moins l'un parmi : les phénols substitués au polyisobutényle, les phénols polyisobutényl amino, l'amine polyisobutényle et leurs mélanges ; les produits de réaction du polyisobutylène avec les oléfines α,β-insaturées suivis d'une autre fonctionnalisation par réaction avec des nucléophiles telles que l'eau, l'alcool, les polyols, les amines, les polyamines, les alkanolamines, et les bases inorganiques ; les produits de réaction du polyisobutylène avec l'acide glyoxylique, les glyoxylates alkyles inférieurs, tels que le méthyl glyoxylate, ou les hémiacétaux alkyles inférieurs, tels que l'hémiacétal de méthyl glyoxylate d'acide méthylique, suivis d'une autre fonctionnalisation avec des amines primaires ou secondaires, des alkanolamines primaires ou secondaires ou des polyamines.

4. Composition selon la revendication 1, dans laquelle le co-émulsifiant comprend un monooléate de sorbitane, un monoisostéarate de sorbitane, un monooléate de glycérol, un 2-éthoxylate d'alcool oléylique, la lécithine, un monolaurate de sorbitane, un monopalmitate de sorbitane, un monostéarate de sorbitane, un sesquistéarate de sorbitane, un trioléate de sorbitane, un 2-éthoxylate d'alcool stéarylique, un monostéarate de glycérol, un dioléate de sorbitane, la lanoline, un dioléate de méthylglucoside, un diisostéarate de polyglycéryl-3, un polyéthylène glycol 200 distéarate, un sesquistéarate de méthylglucose, un polyéthylène glycol 200 monostéarate, des alcoxylates d'alcool, des copolymères de divers alcoxylates, des amines ou des alcools ou des amides alkyles, des amines ou des amides alkyles éthoxylées, des bétaïnes, des sulfonates ou des sulfates ou des sulfosuccinates ou des phosphonates ou des borates ou des sels d'amine ou des carboxylates compatibles, ou leurs mélanges et le co-émulsifiant est présent en une quantité allant de 0 % en poids à 10 % en poids de l'émulsion eau-huile interne.

5. Composition selon la revendication 1, dans laquelle le matériau aqueux comprend de l'eau du robinet, de l'eau déminéralisée, de l'eau déionisée, de l'eau florale ou leurs mélanges ; et
dans laquelle l'huile comprend des substances grasses, des huiles volatiles, des huiles non-volatiles et leurs mélanges ; et
dans laquelle la phase aqueuse externe comprend de l'eau du robinet, de l'eau déminéralisée, de l'eau déionisée, de l'eau florale ou leurs mélanges ;
et l'eau est présente en une quantité allant de 1 % en poids à 99 % en poids de la phase aqueuse interne de l'émulsion aqueuse ; et
l'huile est présente en une quantité allant de 99 % en poids à 1 % en poids de l'émulsion eau-huile ; et
dans laquelle le rapport émulsion eau-huile interne à la phase aqueuse externe se situe dans la plage allant de 10 pour 90 à 90 pour 10.

6. Composition selon la revendication 1, dans laquelle l'émulsifiant de la phase aqueuse externe comprend les éthoxylates, les acides gras éthoxylés non ioniques, les esters, les esters de sorbitane, les alkylphénols, un monolaurate de sorbitane, les carboxylates, les sels amines, les sels métalliques, les alkylarylsulfonates, les oxydes amines, les composés poly(oxyalkylène), comprenant les copolymères séquencés comprenant les unités de répétition d'oxyde d'alkylène, les éthoxylates d'alcool carboxylé, les alcools éthoxylés, les alkylphénols éthoxylés, les amines et les amides éthoxylés, les acides gras éthoxylés, les esters gras et les huiles éthoxylés, les esters gras, les amides d'acides gras, comprenant mais ne se limitant pas aux amides provenant d'acides gras et polyamides de tallöl, esters de glycérol éthoxylés, esters de glycol éthoxylés, esters de sorbitane éthoxylés, dérivés d'imidazoline, lécithine et ses dérivés, lignine et ses dérivés, monoglycérides et leurs dérivés, sulfonates d'oléfine, esters de phosphate et leurs dérivés, acides gras ou alcools ou alkylphénols propoxylés et éthoxylés, dérivés de sorbitane, esters de sucrose et leurs dérivés, sulfates ou alcools ou alcools ou esters gras éthoxylés, sulfonates de dodécyl et tridécyl benzènes ou naphtalènes ou pétrole condensés, sulfosuccinates et leurs dérivés, et acides sulfoniques de benzène tridécyl et dodécyl ou leurs mélanges et est présent en une quantité située dans la plage allant de 0,01 % en poids à 10 % en poids de l'émulsion multiple.

7. Composition selon la revendication 1, dans laquelle l'émulsion multiple est épaissie par un épaississant comprenant de la gomme xanthique, des dérivés cellulosiques, de la chitosane, des amidons, des silicates, des silicates d'aluminium de magnésium, de l'hydroxyéthylcellulose, des gommes xanthiques, des polysaccharides de glucose-mannose, de l'ammonium poly(acryldiméthyltauramde-co-vinylformamide), l'alcool stéarylique, l'alcool cétylique, l'alcool cétéarylique, les argules, les hectorites, les smectites, les bentonites, un copolymère d'acide polyacrylique réticulé, un polymère polyacrylate modifié réticulé, des polyéthoxylates de méthylglucose et leurs dérivés, le PEG-120 méthylglucose dioléate, l'amidon, l'amidon de pomme de terre modifié, les copolymères d'éthylène/propylène/styrène, les copolymères de butylènes/éthylène/styrène, le polyisobutylène, le polyisobutylène hydrogéné, les cires, la cire de polyéthylène, la cire d'abeille, les polyacrylates d'huiles solubles, les polyméthacrylates d'huiles solubles, les polymères oléfiniques, les copolymères oléfiniques, les copolymères oléfiniques fonctionnalisés, les terpolymères oléfiniques, les terpolymères oléfiniques fonctionnalisés, les argiles modifiées de manière hydrophobique, les silices, et les copolymères de styrène et d'oléfines ou leurs mélanges et dans laquelle l'épaississant est présent en une quantité située dans la plage allant de 0 % en poids à 10 % en poids de l'émulsion multiple.

8. Composition selon la revendication 1, dans laquelle la composition comprend des additifs hydrosolubles comprenant le propylène glycol, des agents actifs, des conservateurs, des antioxydants, des agents complexants, des parfums, des matériaux de remplissage, des bactéricides, des absorbeurs d'odeurs, des matières colorantes, des colorants, des liposomes, des hydrolysats de protéine et des polyols tels que la glycérine ; les glycols tels que les polyéthylène glycols ; les dérivés du sucre ; les extraits naturels ; les agents éclaircissants, les agents décolorants, les agents botaniques, des agents regraissants, des revitalisants pour la peau et les cheveux ; des vitamines, l'urée ; la caféine ; des agents dépigmentants tels que l'acide kojique et l'acide caféique ; les acides bêta-hydroxy tels que l'acide salicylique et ses dérivés ; les acides alpha-hydroxy tels que l'acide lactique et l'acide glycolique ; les émollients et les humectants, tels que les méthyl glucosides éthoxylés et les méthyl glucosides acylés éthoxylés ; le dihydroxyacétone, les acides aminés et les mélanges d'acides aminés, les sels inorganiques, les oxydes inorganiques, les écrans solaires, les rétinoïdes tels que le rétinol et ses dérivés et les caroténoïdes ; les agents écrans organiques et inorganiques ; l'hydrocortisone ; la DHEA ; la mélatonine ; les extraits algacés, fongiques, de plantes, de levures ou de bactéries ; les protéines, hydrolysées, partiellement hydrolysées ou non hydrolysées ; les enzymes ou les mélanges de celles-ci et dans laquelle les additifs hydrosolubles sont présents en une quantité située dans la plage allant de 0 % en poids à 30 % en poids de l'émulsion multiple.

9. Composition selon la revendication 1, dans laquelle la composition comprend des additifs dispersibles dans l'eau comprenant des argiles, des pigments, des oxydes d'aluminium, des silicates, du talc, des silicates de magnésium, du dioxyde de titane, de l'oxyde de zinc ou les mélanges de ceux-ci et dans laquelle les additifs dispersibles dans l'eau sont présents en une quantité située dans la plage allant de 0 % en poids à 30 % en poids de l'émulsion multiple.

10. Composition selon la revendication 1, dans laquelle la composition huileuse comprend des additifs en phase huileuse comprenant des additifs lipophobes, des acides gras, des alcools gras, des gommes, des cires, des gommes de silicone, des agents de gélification des huiles, des particules organiques, des particules inorganiques, des épaississants, des vitamines, des écrans totaux organiques, l'avobenzone, l'octocrylène, les esters de cinnamate, les esters de salicylate, des agents regraissants, des revitalisants pour la peau, des revitalisants capillaires, des émollients, des agents hydratants, des lanolines ou leurs mélanges et dans laquelle les additifs en phase huileuse sont présents en une quantité située dans la plage allant de 0 % en poids à 30 % en poids de la phase interne de l'émulsion eau-huile.

11. Composition selon la revendication 1, dans laquelle l'émulsion eau-huile interne dans la phase aqueuse externe est composée de gouttelettes de l'émulsion eau-huile interne ayant un diamètre moyen allant de 1 à 50 µm.

12. Composition selon la revendication 1, dans laquelle l'émulsifiant huile-eau a un HLB allant de 8 à 20.

13. Composition selon la revendication 1, utilisée comme émulsion multiple pour des produits choisis dans le groupe consistant en les produits industriels, les produits ménagers, les produits de consommation, les produits cosmétiques, les produits de travail de métaux, les produits horticoles, les produits agricoles, les produits de revêtement, les produits de peinture, les produits d'encres, les produits lubrifiants, les produits de carburant et leurs combinaisons.

14. Procédé de fabrication d'une composition émulsion multiple comprenant la combinaison d'un composant aqueux dans la plage allant de 1 % en poids à 99 % en poids et une phase huileuse dans la plage allant de 99 % en poids à 1 % en poids avec un émulsifiant choisi dans le groupe comprenant un émulsifiant dérivé d'anhydride poly(isobutényl) succinique, un émulsifiant dérivé de polyisobutylène et leurs mélanges afin de produire une émulsion eau-huile interne, et la combinaison de la phase eau-huile interne dans une phase aqueuse externe à un rapport de 10 pour à 90 à 90 pour 10 avec un émulsifiant ayant un HLB allant de 8 à 20, ce qui résulte en une émulsion multiple stable.
